# EUROPEAN PATENT SPECIFICATION

(11) **EP 3 816 178 B1**
(45) Date of publication and mention of the grant of the patent: **23.11.2022**
(21) Application number: 19799774.5
(22) Date of filing: 07.05.2019
(51) Int. Cl.: C07K 7/08, A23L 33/18, A61K 38/10, A61P 25/24, C07K 14/415, C07K 7/06, A61K 38/00

(54) **PEPTIDE, COMPOSITION, AND METHOD FOR TREATING, PREVENTING, OR AMELIORATING MOOD DISORDER**
PEPTID, ZUSAMMENSETZUNG UND VERFAHREN ZUR BEHANDLUNG, VORBEUGUNG ODER LINDERUNG VON GEMÜTSSTÖRUNGEN
PEPTIDE, COMPOSITION, ET MÉTHODES DE TRAITEMENT, DE PRÉVENTION OU D'ATTÉNUATION D'UN TROUBLE DE L'HUMEUR

(30) Priority: 08.05.2018 JP 2018089784
(43) Date of publication of application: 05.05.2021
(73) Proprietor: Kyoto University, Kyoto-shi, Kyoto 606-8501 (JP); Kazusa DNA Research Institute, Kisarazu-shi, Chiba 292-0818 (JP); Kameda Seika Co., Ltd., Niigata-shi, Niigata 950-0198 (JP)
(72) Inventor: OHINATA Kousaku, Kyoto-shi, Kyoto 606-8501 (JP); ASAKURA Saho, Kyoto-shi, Kyoto 606-8501 (JP); SUZUKI Hideyuki, Kisarazu-shi, Chiba 292-0818 (JP); SATO Masaru, Kisarazu-shi, Chiba 292-0818 (JP); ITO Akira, Niigata-shi, Niigata 950-0198 (JP); HIGUCHI Yuki, Niigata-shi, Niigata 950-0198 (JP)
(74) Representative: Maiwald GmbH
(86) International application number: PCT/JP2019/018229
(87) International publication number: WO 2019/216307

(56) References cited:
- WO-A1-2013/129220
- JP-A- 2018 002 616
- JP-A- 2018 002 616
- MORITANI CHIE ET AL: "Isolation of activating factors of serotoninN-acetyltransferase from rice peptides", JOURNAL OF FUNCTIONAL FOODS, ELSEVIER BV, NL, vol. 41, 26 December 2017 (2017-12-26), pages 148-154, XP085349202, ISSN: 1756-4646, DOI: 10.1016/J.JFF.2017.12.051
- MCCLUNG COLLEEN A ED - SANACORA GERARD ET AL: "How Might Circadian Rhythms Control Mood? Let Me Count the Ways", BIOLOGICAL PSYCHIATRY, ELSEVIER SCIENCE, NEW YORK, NY; US, vol. 74, no. 4, 1 April 2013 (2013-04-01), pages 242-249, XP028680322, ISSN: 0006-3223, DOI: 10.1016/J.BIOPSYCH.2013.02.019
- COLLEEN A MCCLUNG ED - FINEBERG N A ET AL: "Circadian rhythms and mood regulation: Insights from pre-clinical models", EUROPEAN NEUROPSYCHOPHARMACOLOGY, ELSEVIER SIENCE PUBLISHERS BV , AMSTERDAM, NL, vol. 21, 11 August 2011 (2011-08-11), pages S683-S693, XP028292114, ISSN: 0924-977X, DOI: 10.1016/J.EURONEURO.2011.07.008 [retrieved on 2011-07-20]
- Masaaki Ikeda: "Effects of moodstabilizer on the circadian system: possible implication for abnormalities in mood disorders.", Folia Pharmacologica Japonica, vol. 130, no. 6, 2007, pages 469-476, XP055759075, ISSN: 0015-5691, DOI: 10.1254/fpj.130.469

## Description

### TECHNICAL FIELD

The present invention relates to a peptide, a composition comprising said peptide, and their use for treating, preventing or ameliorating a mood disorder, as defined in the claims.

### BACKGROUND ART

Reflecting the modern stress society, an increase in mood disorders represented by reduced motivation and depression etc., is a problem. Anxiety, which is one of the etiologies of mood disorders, is originally essential as a warning to avoid risks in organism, but excessive anxiety is involved in the onset of mood disorders and the progression of the symptoms. The development of foods and pharmaceutical products to alleviate anxiety is expected.

For example, Patent Document 1 describes that a predetermined dipeptide is suitable as an anxiolytic drug or the like.

Patent Document 2 describes a NAT (serotonin-N-acetyltransferase) activating agent peptide of sequence YQQQFQQFLPEGQSQSQK which is suitable to improve sleep and depression.

Non-Patent Document 1 describes activating factors of serotonin-N-acetyltransferase isolated from rice peptides. One of them has the sequence YQQQFQQFLPEGQSQSQK and discusses the effects of starchy foods on sleep.

Patent Document 1: PCT International Publication No. WO2013/129220

Patent Document 2: JP 2018 002616 A

Non-Patent-Document 1: Moritani Chie et al., Journal of Functional Foods, vol. 41 (2017), pages 148-154

### DISCLOSURE OF THE INVENTION

### Problems to be Solved by the Invention

However, there is a further need for functional materials capable of treating, preventing, or ameliorating mood disorders.

The present invention has been achieved in view of the above circumstances, and an object thereof is to provide a novel peptide capable of treating, preventing or ameliorating mood disorders.

### Means for Solving the Problems

The present inventors have discovered that the above problems can be solved by using a peptide consisting of a specific amino acid sequence, and have completed the present invention. Specifically, the present invention provides the following.
(1) A peptide comprising an amino acid sequence represented by SEQ ID NO: 1 and having an amino acid length of 6 or more and 13 or less.
(2) The peptide according to (1), wherein the peptide consists of the amino acid sequence represented by SEQ ID NO: 1.
(3) The peptide according to (1), wherein the peptide consists of an amino acid sequence represented by SEQ ID NO: 2 or an amino acid sequence having 90% or more identity with the amino acid sequence represented by SEQ ID NO: 2.
(4) The peptide according to (3), wherein the peptide consists of the amino acid sequence represented by SEQ ID NO: 2.
(5) The peptide according to any one of (1) to (4), for use in treating, preventing, or ameliorating a mood disorder.
(6) The peptide for use according to (5), wherein the mood disorder is one or more disorders selected from the group consisting of reduced motivation, depression, and depressive mood disorder.
(7) A composition, which comprises the peptide according to any one of (1) to (4).
(8) The composition according to (7), which is a pharmaceutical product.
(9) The composition according to (7), which is a food or drink.
(10) The composition according to any one of (7) to (9) for use in treating, preventing, or ameliorating a mood disorder.
(11) The composition for use according to (10), wherein the mood disorder is one or more disorders selected from the group consisting of reduced motivation, depression, and depressive mood disorder.

### Effects of the Invention

According to the present invention, a novel peptide capable of treating, preventing or ameliorating mood disorders is provided.

### BRIEF DESCRIPTION OF THE DRAWINGS

Fig. 1 shows the results of a tail suspension test using mice orally administered with the peptide of the present invention;
Fig. 2 shows the results of a tail suspension test using mice orally administered with the peptide of the present invention; and
Fig. 3 shows the results of a tail suspension test using mice orally administered with the peptide of the present invention.

### PREFERRED MODE FOR CARRYING OUT THE INVENTION

Hereinafter, embodiments of the present invention will be described in detail.

### <Peptide of the present invention>

The peptide of the present invention comprises the amino acid sequence (QSQSQK) represented by SEQ ID NO: 1 and has an amino acid length of 6 or more and 13 or less. Hereinafter, amino acid sequences are each described from the N-terminus on the left end to the C-terminus.

As a result of studies based on simultaneous analysis information of various peptide mixtures and structure-activity correlation information of known peptides that affect emotional behavior, the present inventors have discovered that a novel peptide exhibiting effects on treatment of mood disorders, that is, the above peptide.

As a result of further studies, the present inventors have discovered that the peptide of the present invention brings about a motivation-improving effect by activating the dopamine D1 receptor, and can exhibit an effect of treating or preventing mood disorders.

The peptide of the present invention may consist of the amino acid sequence represented by SEQ ID NO: 1, or may be prepared by adding any amino acid to the N-terminal side and/or the C-terminal side of the amino acid sequence represented by SEQ ID NO: 1. In the amino acid sequence represented by SEQ ID NO: 1, the N-terminal amino acid is Q (glutamine) and the C-terminal amino acid is K (lysine).

The upper limit of the amino acid length of the present invention is 13 amino acids or less, and preferably 6 amino acids (that is, the peptide consists of the amino acid sequence represented by SEQ ID NO: 1). The peptide of the present invention tends to easily exhibit the effects of the present invention as the amino acid length is shorter.

The peptide prepared by adding an amino acid to the N-terminal side and/or the C-terminal side of the amino acid sequence represented by SEQ ID NO: 1 is not particularly limited, but an example thereof consists of the amino acid sequence represented by SEQ ID NO: 2 (QQFLPEGQSQSQK) or an amino acid sequence having 90% or more identity with the amino acid sequence represented by SEQ ID NO: 2. Note that the amino acid sequence represented by SEQ ID NO: 2 has 7 amino acids (QQFLPEG) added to the N-terminal side of the amino acid sequence represented by SEQ ID NO: 1.

The peptide of the present invention may be, for example, a peptide prepared by substitution of one amino acid in the amino acid sequence represented by SEQ ID NO: 2 with any amino acid. The peptide prepared by adding an amino acid to the N-terminal side and/or C-terminal side of the amino acid sequence represented by SEQ ID NO: 1 may consist of the amino acid sequence represented by SEQ ID NO: 2 (QQFLPEGQSQSQK) or an amino acid sequence having 90% or more identity with the amino acid sequence represented by SEQ ID NO: 2.

The peptide of the present invention can be obtained by chemical synthesis or hydrolysis of a natural protein or polypeptide.

Examples of chemical synthesis methods include known peptide synthesis methods. Specific examples thereof include a liquid phase method or a solid phase method which is a method usually employed for peptide synthesis. More specific examples thereof include the Fmoc method and the Boc method. The synthesized peptide may also be purified. Examples of the purification method include methods using ion exchange chromatography, reverse phase liquid chromatography, and affinity chromatography.

Examples of a hydrolysis method include a method using hydrolase and a method using a strong acid or a strong base.

In the method using hydrolase, hydrolase derived from an animal, a plant or a microorganism (trypsin, chymotrypsin, papain, pepsin, carboxypeptidase, thermolysin) can be used. As the hydrolase, microorganisms (for example, edible yeasts such as baker's yeast and brewer's yeast) that can be used as foods may also be used.

Conditions for hydrolysis using hydrolase are not particularly limited, but the pH is adjusted to an appropriate value according to the enzyme used, and the reaction may be performed at a temperature between 30°C and 40°C for 30 minutes to 48 hours. The peptide of the present invention may be purified from the thus obtained reaction liquid and then used. When a food material is subjected to hydrolysis, the food material can be used intact or can be added to another food material(s) and then the product can be served as a food.

In a method using a strong acid, for example, hydrochloric acid, nitric acid, and sulfuric acid can be used. In a method using a strong base, for example, alkali metal hydroxides (sodium hydroxide, potassium hydroxide, lithium hydroxide), alkali metal carbonates (sodium carbonate, potassium carbonate), and alkali metal bicarbonates (sodium hydrogen carbonate, potassium hydrogen carbonate) can be used.

Conditions for hydrolysis using a strong acid or a strong base are not particularly limited, but the reaction may be performed in water at a temperature between 1 and 100°C for 30 minutes to 48 hours in the presence of a strong acid or a strong base. The reaction product of hydrolysis may be used intact after adjusting the pH, or the peptide of the present invention may be isolated by purification and then used.

The amino acid sequences of peptides obtained by various methods can be analyzed using a protein sequencer that reads an amino acid sequence from the C-terminus by Edman degradation, or GC-MS.

### <Composition of the present invention>

The composition of the present invention comprises at least the peptide of the present invention, may consist of the peptide of the present invention, and may comprise other components.

When such peptide to be comprised in the composition comprises an amino acid sequence other than the peptide of the present invention, as a portion of the sequence, the effects of the present invention tend to be easily exhibited as the sequence length is shorter.

Mood disorders can be treated, prevented or ameliorated by ingesting the peptide of the present invention. Therefore, the composition of the present invention can be preferably used for treating, preventing or ameliorating mood disorders.

In the present invention, "mood disorders" refers to a mental illness having a disorder related to mood (emotion). Specific examples thereof include decreased motivation, depression, and depressive mood disorder, and one or more symptoms based on them. According to the present invention, it is possible to treat, prevent or ameliorate particularly reduced motivation among mood disorders.

In the present invention, "treatment" refers to, for example, delaying the progression of mood disorders and healing of the symptoms. "Prevention" refers to, for example, suppression or delay of the onset of mood disorders. "Amelioration" refers to, for example, alleviation or relief of the symptoms of mood disorders.

The composition of the present invention can be prepared in any form, and may be prepared as a pharmaceutical product or a food or drink.

When the composition of the present invention is prepared as a pharmaceutical product, it can be prepared as an oral administration agent or a parenteral administration agent. The composition of the present invention can be prepared as the following preparations containing the peptide of the present invention alone or the peptide together with a carrier, a diluent or an excipient: tablets (plain tablets, sugar-coated tablets, effervescent tablets, film-coated tablets, chewable tablets), capsules, troches, powders, fine granules, granules, solutions, suspensions, emulsions, pastes, creams, injections (including cases where they are blended into infusions such as amino acid infusions and electrolyte infusions), enteric-coated tablets, capsules, and sustained-release preparations.

As the carrier, diluent or excipient, a substance which is commonly used in the pharmaceutical field and does not react with the peptide of the present invention is used. Examples thereof include: lactose, glucose, mannitol, dextrin, cyclodextrin, starch, sucrose, magnesium aluminate metasilicate, synthetic aluminum silicate, sodium carboxymethylcellulose, hydroxypropyl starch, carboxymethylcellulose calcium, ion exchange resin, methylcellulose, gelatin, gum arabic, hydroxypropylcellulose, hydroxypropylmethylcellulose, polyvinylpyrrolidone, polyvinyl alcohol, light anhydrous silicic acid, magnesium stearate, talc, tragacanth, bentonite, veegum, titanium oxide, sorbitan fatty acid ester, sodium lauryl sulfate, glycerin, fatty acid glycerin ester, purified lanolin, glycerogelatin, polysorbate, macrogol, vegetable oils, waxes, liquid paraffin, white petrolatum, fluorocarbons, nonionic surfactants, propylene glycol, and water.

When the composition of the present invention is prepared as a food or drink, it can be prepared in any form, and examples thereof include the following: beverages (coffee, cocoa, juice, soft drink, mineral drink, tea drink, green tea, black tea, oolong tea, milk drink, lactobacillus beverage, yogurt drink, carbonated drink), gum, gummy, jelly, candy, cookies, crackers, biscuits, ice confectioneries (ice cream, ice candy, sorbet, shaved ice), retort foods, and jelly foods (jelly, agar, jelly-like beverage).

The foods or drinks of the present invention may also be prepared as so-called health foods, functional foods, dietary supplements, supplements, foods for specified health use, functional indication foods, foods for sick people and combination foods for sick people (Ministry of Health, Labor and Welfare, a kind of special-purpose foods) or foods for the elderly (Ministry of Health, Labor and Welfare, a kind of special-purpose food).

The amount of the peptide of the present invention in the composition of the present invention can be appropriately set according to the effects to be obtained. For example, the peptide of the present invention may be blended in an amount of preferably 0.01 mass% or more, more preferably, 1.00 mass% or more relative to the composition. Further, the peptide of the present invention may be blended in an amount of preferably 100 mass% or less, more preferably 90 mass% or less relative to the composition. Note that the above values are expressed in terms of the amount of the peptide of the present invention, when peptides other than the peptide of the present invention are contained in the composition of the present invention.

The amounts of components other than the peptide of the present invention in the composition of the present invention can be appropriately set according to the types of the components, the form of the composition, and the effects to be obtained.

The administration method of the composition of the present invention is not particularly limited, and may be either oral administration or parenteral administration (injection). From the viewpoint that the effects of the present invention tend to be easily exhibited, the composition of the present invention is preferably administered orally.

The dose of the composition of the present invention varies depending on the administration method, and the conditions and age of the administration subject. For example, in terms of the amount of the peptide of the present invention, the dose ranges from preferably 0.01 mg/kg to 500 mg/kg, more preferably 0.05 mg/kg to 100 mg/kg, and still more preferably 0.1 to 30 mg/kg per day for an adult. Within the above range, the effects of the present invention tend to be more easily exhibited as the dose is increased.

As the method for producing the composition of the present invention, a known method can be employed depending on the form to be obtained.

### <Treating, preventing or ameliorating mood disorders>

Through administration of the peptide or the composition of the present invention to a subject, mood disorders can be treated, prevented or ameliorated.

The administration method can be appropriately selected depending on the form of the composition.

The number of administrations, the administration interval, and the dose can be appropriately selected according to the conditions (symptom, age, body weight) of the administration subject.

The administration subject is not particularly limited, and examples thereof include humans and non-human mammals (dogs, cats, livestock (such as cattle, pigs, sheep, goats)).

### EXAMPLES

Hereinafter, the present invention will be specifically described with reference to examples. However, the present invention is not limited to these examples.

### <Preparation of the peptide of the present invention>

The following two types of the peptide of the present invention were prepared through synthesis by the Fmoc method and then purification by reverse phase HPLC.
(1) Peptide consisting of the amino acid sequence (QSQSQK) represented by SEQ ID NO: 1
(2) Peptide consisting of the amino acid sequence (QQFLPEGQSQSQK) represented by SEQ ID NO: 2

Further, in the amino acid sequence represented by SEQ ID NO: 2, a peptide consisting of 7 residues; that is, a half of the N-terminal portion (peptide consisting of the amino acid sequence (QQFLPEG) represented by SEQ ID NO: 3), was prepared in the same manner as described above.

Each prepared peptide was administered to a mouse (ddy mouse (5-week-old male, body weight 24g-28 g)) in each of the following tests, and then the motivation-improving effect was evaluated by a tail suspension test.

### <Evaluation of motivation-improving effect: Tail suspension test>

Each mouse was suspended by the tail and hung 30 cm above the floor, 30 minutes after peptide administration. Subsequently, over a period of 6 minutes from the start of the test (0 minute), the time (immobility time) during which the mouse was in an immobility state after initiating escape behavior was measured. The immobility state is known as a "despair state", and it can be evaluated that the shorter the immobility time, the better the despair state and the greater the motivation. Therefore, substances that provide a motivation-improving effect in this test can be effective in treating, preventing, or ameliorating mood disorders.

### <Test 1>

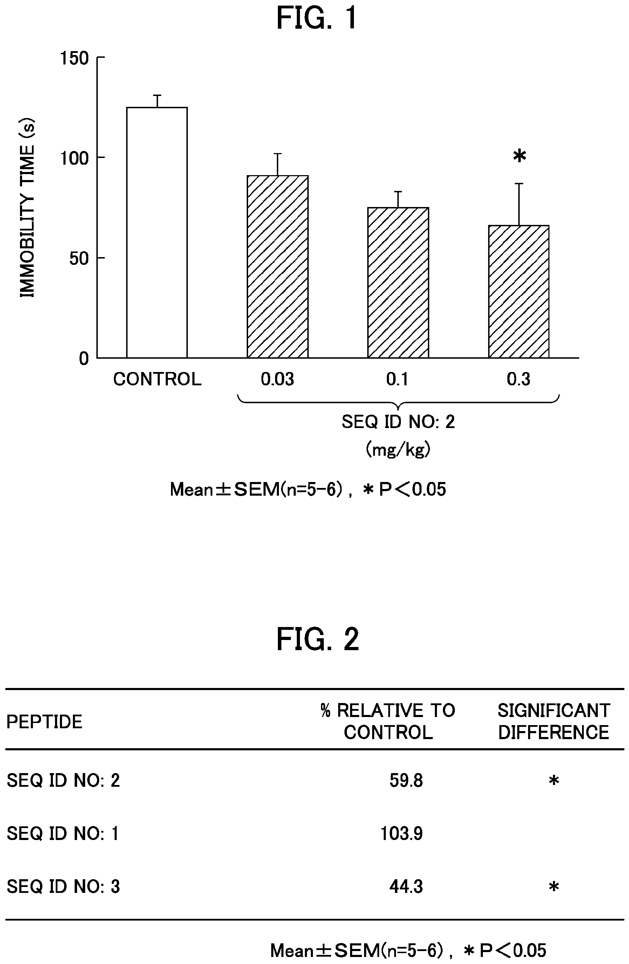

The amino acid sequence represented by SEQ ID NO: 2 was dissolved in physiological saline and orally administered to each mouse in an amount of 0.03, 0.1, or 0.3 mg/kg per body weight (n = 5 to 6). As a control, mice were prepared by orally administering physiological saline alone (n = 6). Each mouse was subjected to a tail suspension test, and the motivation-improving effect resulting from the peptide of the present invention was evaluated. The results are shown in Fig. 1.

As shown in Fig. 1, the mice to which the peptide of the present invention had been administered had the immobility time shorter than that of the control. This tendency was recognized depending on the concentration of the peptide of the present invention. Therefore, it was demonstrated that the peptide of the present invention exhibits the motivation-improving effect and is useful for treatment and prevention of mood disorders.

### <Test 2>

Each of the amino acid sequences represented by SEQ ID NOS: 1 to 3 was dissolved in physiological saline and orally administered to mice in an amount of 0.1 mg/kg per body weight (n = 5 to 6). As a control, mice were prepared by orally administering physiological saline alone to the mice (n = 6). Each mouse was subjected to a tail suspension test, and the motivation-improving effect resulting from the peptide of the present invention was evaluated. The results are shown in Fig. 2.

As shown in FIG. 2, the mice to which the amino acid sequence represented by SEQ ID NO: 3 had been administered were not observed to have any decrease in immobility time compared to the control. On the other hand, the mice to which the amino acid sequence represented by SEQ ID NO: 1 or 2 had been administered had a shorter immobility time than the control. From these results, it was found that the peptide containing the amino acid sequence of the amino acid sequence represented by SEQ ID NO: 1 in its sequence has a significant motivation-improving effect.

### <Test 3>

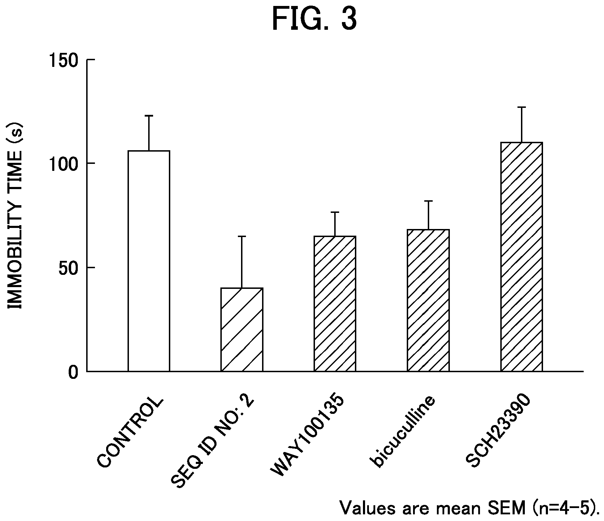

Mice were prepared by orally administering the amino acid sequence represented by SEQ ID NO: 2 in an amount of 0.3 mg/kg and various receptor antagonists in combination thereto (n = 5 to 6). Each mouse was subjected to a tail suspension test, and the motivation-improving effect resulting from the peptide of the present invention was evaluated. The results are shown in Fig. 3.

The following antagonists were used.
(1) SCH23390: dopamine D1 receptor antagonist, and the amount used: 30 µg/kg
(2) WAY100135: serotonin 5-HT1A receptor antagonist, and the amount used: 10 mg/kg
(3) Bicuculline: GABA-A receptor antagonist, and the amount used: 30 mg/kg

In Fig. 3, the longer the immobility time, the more the receptor inhibited by the antagonist contributes to the motivation-improving effect resulting from the amino acid sequence represented by SEQ ID NO: 2. The combined use of the amino acid sequence represented by SEQ ID NO: 2 and WAY100135 or Bicuculline relatively decreased the immobility time. This means that serotonin 5-HT1A receptor and GABA-A receptor are almost unrelated to the motivation-improving effect resulting from the amino acid sequence represented by SEQ ID NO: 2. On the other hand, the combined use of the amino acid sequence represented by SEQ ID NO: 2 and SCH23390 resulted in significantly increased immobility time. These results suggested that the activation of the dopamine D1 receptor may contribute to the motivation-improving effect resulting from the amino acid sequence represented by SEQ ID NO: 2.

## Claims

1. A peptide comprising an amino acid sequence represented by SEQ ID NO: 1 and having an amino acid length of 6 or more and 13 or less.

2. The peptide according to claim 1, wherein the peptide consists of the amino acid sequence represented by SEQ ID NO: 1.

3. The peptide according to claim 1, wherein the peptide consists of an amino acid sequence represented by SEQ ID NO: 2 or an amino acid sequence having 90% or more identity with the amino acid sequence represented by SEQ ID NO: 2.

4. The peptide according to claim 3, wherein the peptide consists of the amino acid sequence represented by SEQ ID NO: 2.

5. The peptide according to any one of claims 1 to 4, for use in treating, preventing, or ameliorating a mood disorder.

6. The peptide for use according to claim 5, wherein the mood disorder is one or more disorders selected from the group consisting of reduced motivation, depression, and depressive mood disorder.

7. A composition which comprises the peptide according to any one of claims 1 to 4.

8. The composition according to claim 7, which is a pharmaceutical product.

9. The composition according to claim 7, which is a food or drink.

10. The composition according to any one of claims 7 to 9, for use in treating, preventing, or ameliorating a mood disorder.

11. The composition for use according to claim 10, wherein the mood disorder is one or more disorders selected from the group consisting of reduced motivation, depression, and depressive mood disorder.

## Patentansprüche

1. Ein Peptid umfassend eine Aminosäuresequenz repräsentiert durch SEQ ID NO: 1 und mit einer Länge von 6 oder mehr und 13 oder weniger Aminosäuren.

2. Das Peptid gemäß Anspruch 1, wobei das Peptid aus der Aminosäuresequenz repräsentiert durch SEQ ID NO: 1 besteht.

3. Das Peptid gemäß Anspruch 1, wobei das Peptid aus einer Aminosäuresequenz besteht, die durch SEQ ID NO: 2 oder durch eine Aminosäuresequenz mit 90% oder mehr Identität mit der Aminosäuresequenz repräsentiert durch SEQ ID NO: 2 repräsentiert wird.

4. Das Peptid gemäß Anspruch 3, wobei das Peptid aus der Aminosäuresequenz repräsentiert durch SEQ ID NO: 2 besteht.

5. Das Peptid gemäß irgendeinem der Ansprüche 1 bis 4 zur Verwendung in der Behandlung, Vorbeugung, oder Linderung einer seelischen Erkrankung.

6. Das Peptid zur Verwendung gemäß Anspruch 5, wobei die seelische Erkrankung eine oder mehrere Erkrankungen ausgewählt au der Gruppe bestehend aus Antriebslosigkeit, Depression, und depressiver Verstimmung ist.

7. Eine Zusammensetzung umfassend das Peptid gemäß irgendeinem der Ansprüche 1 bis 4.

8. Die Zusammensetzung gemäß Anspruch 7, welche ein Arzneimittel ist.

9. Die Zusammensetzung gemäß Anspruch 7, welche ein Nahrungsmittel oder ein Getränk ist.

10. Die Zusammensetzung gemäß irgendeinem der Ansprüche 7 bis 9 zur Verwendung in der Behandlung, Vorbeugung, oder Linderung einer seelischen Erkrankung.

11. Die Zusammensetzung zur Verwendung gemäß Anspruch 10, wobei die seelische Erkrankung eine oder mehrere Erkrankungen ausgewählt au der Gruppe bestehend aus Antriebslosigkeit, Depression, und depressiver Verstimmung ist.

## Revendications

1. Peptide comprenant une séquence d'acides aminés représentée par SEQ ID NO : 1 et ayant une longueur d'acides aminés de 6 ou plus et de 13 ou moins.

2. Peptide selon la revendication 1, le peptide consistant en la séquence d'acides aminés représentée par SEQ ID NO : 1.

3. Peptide selon la revendication 1, le peptide consistant en une séquence d'acides aminés représentée par SEQ ID NO : 2 ou une séquence d'acides aminés ayant 90 % ou plus d'identité avec la séquence d'acides aminés représentée par SEQ ID NO : 2.

4. Peptide selon la revendication 3, dans lequel le peptide consiste en la séquence d'acides aminés représentée par SEQ ID NO : 2.

5. Peptide selon l'une quelconque des revendications 1 à 4, destiné à être utilisé dans le traitement, la prévention ou l'amélioration d'un trouble de l'humeur.

6. Peptide destiné à être utilisé selon la revendication 5, dans lequel le trouble de l'humeur est un ou plusieurs troubles sélectionnés dans le groupe consistant en une motivation réduite, une dépression et un trouble dépressif de l'humeur.

7. Composition qui comprend le peptide selon l'une quelconque des revendications 1 à 4.

8. Composition selon la revendication 7, qui est un produit pharmaceutique.

9. Composition selon la revendication 7, qui est un aliment ou une boisson.

10. Composition selon l'une quelconque des revendications 7 à 9, destinée à être utilisée dans le traitement, la prévention ou l'amélioration d'un trouble de l'humeur.

11. Composition pour une utilisation selon la revendication 10, dans laquelle le trouble de l'humeur est un ou plusieurs troubles sélectionnés dans le groupe consistant en une motivation réduite, une dépression et un trouble de l'humeur dépressif.
